# EUROPÄISCHE PATENTSCHRIFT

(11) **EP 2 797 640 B1**
(45) Veröffentlichungstag und Bekanntmachung des Hinweises auf die Patenterteilung: **20.07.2016**
(21) Anmeldenummer: 12823134.7
(22) Anmeldetag: 19.12.2012
(51) Int. Cl.: A61L 27/18, A61L 27/34, A61L 27/50, A61F 2/06

(54) **STRUKTUR MIT STELLENWEISE MITEINANDER VERKLEBTEN FASERN**
FIBROUS STRUCTURE
STRUCTURE FIBREUSE

(30) Priorität: 29.12.2011 DE 102011122490; 03.05.2012 DE 102012008656
(43) Veröffentlichungstag der Anmeldung: 05.11.2014
(73) Patentinhaber: NonWoTecc Medical GmbH, 50829 Köln (DE)
(72) Erfinder: CLASSEN, Christoph, 52156 Monschau (DE); FRANZEN, Swen, 50935 Köln (DE); VAN BAARS, Harrie, 7552 ED Hengelo (NL); WITT, Wolfgang, 47443 Moers (DE); HENSELER, Andreas, 52152 Simmerath (DE); WILLEMS, Frank, 47441 Moers (DE)
(74) Vertreter: KIPA AB
(86) Internationale Anmeldenummer: PCT/DE2012/001211
(87) Internationale Veröffentlichungsnummer: WO 2013/097841

(56) Entgegenhaltungen:
- EP-A2- 0 237 037
- EP-A2- 2 042 203
- WO-A1-2008/049386
- WO-A1-2011/054932
- WO-A2-02/49536
- DE-A1-102009 037 134
- US-A1- 2002 040 247

## Beschreibung

Die Erfindung betrifft eine Struktur mit stellenweise miteinander verklebten Fasern, die eine Durchlässigkeit für Luft zwischen 0,5 ml/min*cm² und 1,5 ml/min*cm² aufweisen.

Derartige Vliese werden als medizinische Vliese eingesetzt und können plan, gebogen, flexibel oder insbesondere auch zylinderförmig sein. Je nach Ausführungsform sind sie dazu geeignet, als Implantat dauerhaft im menschlichen Körper eingesetzt zu werden. Dies trifft insbesondere auf Vliese aus biokompatiblem Polyurethan zu, die z.B. in röhrenförmiger Gestalt als Gefäßprothese eingesetzt werden können. In diesem Fall kann das Vliesmaterial dazu verwendet werden, eine Rohrwandung zu bilden, durch die eine Kanalisierung von Blut zur Rekonstruktion von degenerierten Blutgefäßen möglich wird. Auf die EP 2 083 750 A1 und die WO 2011 054 932 A1, die derartige Vliese beschreiben, wird vollinhaltlich Bezug genommen.

Das Material zur Herstellung von Gefäßprothesen muss durch seine Eigenschaften viele Anforderungen erfüllen, um dauerhaft zur Bildung eines künstlichen Blutgefäßes verwendet werden zu können. Neben der ausreichenden mechanischen und biologischen Stabilität ist dies vor allem auch die biologische Verträglichkeit des Materials, welche eine Grundvorrausetzung für die Eignung zur Herstellung eines implantierbaren Medizinproduktes ist.

Eine Gefäßprothese sollte eine zuverlässige und dauerhafte Bahnung des Blutstroms gewährleisten, ohne dass es im Laufe der Anwendung zu Undichtigkeiten oder Verschlüssen des Prothesenlumens kommt.

Sieht man von Rupturen als Ursache von Undichtigkeiten ab, ist es im Wesentlichen das Material der Gefäßprothese selber, welches eine Leckage von Blut erzeugen kann. Beispielhaft sei hier die Verwendung eines gewebten Textils als Prothesenwandmaterial erwähnt, welches aufgrund seiner porösen Struktur keine zuverlässige Abdichtung gegen Blutverlust aufweist. Nach Implantation kommt es regelmäßig zu Blutungskomplikationen, die erst nach einer längeren Zeit durch Einsetzen der Blutgerinnung und der dadurch verursachten Abdichtung der Poren reduziert wird. Um diesen Prozess zu beschleunigen werden für solche Materialien Hilfsmittel in Form eines Dichtungsmaterials eingesetzt, um die starken Blutungskomplikationen zu reduzieren. Ein derartiges Material wird in der DE 600 03 046 T2 beschrieben.

Die in der vorliegenden Erfindung verwendete Struktur besitzt konstruktionsbedingt eine derart geringe Porosität, dass es nach Eröffnung der Blutbahn nicht zu einer Blutung durch die Prothese kommt. Die Durchlässigkeit ist so gewählt, dass die in die Struktur und insbesondere in ein Vlies eindringenden Blutzellen sofort die Poren des Materials verstopfen. Zudem kommt es zu einer Gerinnungsreaktion in dem Material, welche zusätzlich die Gefäßwandung abdichtet.

Verschlüsse des Prothesenlumens sind im Allgemeinen biologische Reaktionen auf das Fremdmaterial. Diese Verschlüsse werden im Wesentlichen verursacht durch Hyperplasie (überschießendes Zellwachstum) an den Anastomosen oder einer Thrombose in der Blutbahn.

In der Wissenschaft wird als Hauptursache für das Auftreten einer Hyperplasie an den Anastomosen ein Compliance-Mismatch genannt. Dieses Merkmal tritt dann in Erscheinung, wenn sich die Dehneigenschaften des Prothesenmaterials signifikant von denen des nativen Gefäßes unterscheiden. Die bei dieser Erfindung verwendete Struktur besitzt insbesondere als Vliesmaterial eine ähnliche Compliance wie eine menschliche Arterie.

Eine Thrombose im Lumen einer Gefäßprothese entsteht überwiegend aufgrund einer Aktivierung der Thrombozyten an dem Fremdmaterial. Dabei können das Material selber oder seine Oberflächenstruktur mit den damit verbundenen Strömungsphänomenen an der Oberfläche der Auslöser einer solchen Aktivierung sein.

Ziel der Bereitstellung eines optimalen Materials für eine Gefäßprothese oder eine flächige Abdichtung eines Blutraumes ist es also, dass das Material den Blutzellen gegenüber nicht als störendes Fremdmaterial erscheint. Dazu werden verschiedene technologische Ansätze verfolgt.

Das medizinische Vlies der Firma Nonwotecc, das in der EP 2 083 750 A1 und der WO 2011 054 932 A1 beschrieben ist, besitzt strukturelle Merkmale einer extrazellulären Matrix, so dass sich auf der Innenoberfläche einer aus diesem Material hergestellten Prothese Endothelzellen ansiedeln und verbreiten können. Die Folge dieses Zellwachstums ist die Ausbildung einer luminalen Endothelzellschicht, die das darunterliegende Prothesenmaterial vollständig verdeckt, und somit die Gerinnungsaktivierung im Blut deutlich reduziert.

Das zur Herstellung des Vlieses verwendete Grundmaterial besitzt sehr gute biokompatible Eigenschaften, welche nur eine minimale Reizung der Thrombozyten hervorruft.

Letztlich sind die elastischen Eigenschaften der Struktur und insbesondere des Vliesmaterials derart dimensioniert, dass die daraus hergestellte Gefäßprothese eine dem natürlichen arteriellen Gefäß ähnliche Compliance aufweist.

Ausgehend hiervon liegt der Erfindung die Aufgabe zu Grunde, eine unerwünschte Zellanlagerung an der Struktur zu vermeiden und bei der Verwendung als Gefäßprothese die Offenheitsrate des künstlichen Gefäßes zu erhöhen.

Dies wird durch eine mindestens einseitige Beschichtung erzielt, die die Durchlässigkeit der Struktur auf unter 0,2 ml/min*cm² reduziert.

Somit wird eine relativ dichte Membran beschichtet, obwohl zur Erhöhung der Dichtheit eine Beschichtung nicht mehr notwendig wäre und eine poröse Oberfläche gut für das Zellwachstum wäre.

Zur Bestimmung der Permeabilität/Porosität des Vlieses wird eine definierte Fläche des Materials mit einem definierten Luftdruck beaufschlagt und anschließend der durch das Material strömende Luftvolumenstrom gemessen. Dadurch ergibt sich eine spezielle Porosität, die auch unabhängig von der Meßmethode beispielsweise als Luftwiderstandswert angegeben werden kann.

Vorteilhaft ist es, wenn die Fasern aus Polyurethan bestehen.

Insbesondere zur Herstellung künstlicher Gefäße kann die Struktur ein Vlies aufweisen wie es beispielsweise in der WO 2008/049386 A1 beschrieben ist.

Eine vorteilhafte Ausführungsvariante sieht vor, dass die Fasern zu einem Zylinder verklebt sind und die Beschichtung an der Zylinderinnenseite angeordnet ist. Diese Beschichtung glättet abstehende, ggf. gebrochene Fasern, die zu einer Aktivierung der Thrombozyten im vorbeiströmenden Blut führen können (Thrombenbildung.) Außerdem wird das Eindringen von Wachstumsfaktoren aus dem umliegenden Wundbereich vermindert, um dadurch die Gefahr einer Hyperplasie im Anastomosenbereich zu reduzieren.

Die Beschichtung der Innenoberfläche einer Gefäßprothese aus einem medizinischen Vlies ermöglicht überaschenderweise eine weitere Erhöhung der Offenheitsrate.

Erste Untersuchungen lassen vermuten, dass die Wahrscheinlichkeit einer dauerhaften Offenheit der Prothese dann am größten ist, wenn es zunächst nach dem initialen Blutkontakt mit der Protheseninnenoberfläche zu einer möglichst geringen Fremdkörperreaktion kommt. Nach dieser ersten Kontaktzeit sollte eine dauerhafte autologe Beschichtung der Materialoberfläche von statten gehen, die die Innenoberfläche der Prothese möglichst als körpereigenes Gewebe darstellen lässt. Eine solche autologe Beschichtung besteht im optimalen Fall aus einer geschlossenen Endothelzellschicht.

Vorteilhaft ist es, wenn die Beschichtung Gelatine, Kollagene und/oder Fibrin aufweist. Insbesondere Gelatine kann nach gezielt einzustellenden Zeiträumen resorbiert werden, um eine Anlagerung von Endothelzellen auf der darunterliegenden Faserstruktur ermöglichen zu können.

Eine Beschichtung aus Gelatine, Fibrin und/oder Kollagen ebnet die mikroskopisch raue Oberfläche des Vliesmaterials und reduziert damit die strömungsbedingte Aktivierung der Thrombozyten. Nach einer definierten Zeit, die durch die Herstellung dieser Beschichtung eingestellt werden kann, wird die Beschichtung im Blutstrom resorbiert und legt nach und nach die darunterliegende Vliesstruktur frei, auf die sich nun die Endothelzellen ansiedeln können.

Außerdem wird durch eine hier beschriebene Beschichtung auch das Risiko einer Hyperplasie reduziert, da es zu einer verstärkten Abdichtung der Gefäßinnenseite nach außen kommt, die das Einströmen von Wachstumsfaktoren aus dem das Gefäß umgebenden Wundgebiet verhindert. Diese Wachstumsfaktoren haben offensichtlich einen fördernden Effekt auf die Entstehung einer Hyperplasie im Lumen der Prothese.

Die Unterdrückung einer Hyperplasie kann zusätzlich durch das Einbinden von arzneilichen Wirkstoffen in die Beschichtung erreicht werden, die zeitgesteuert durch die Resorption der Beschichtung freigesetzt werden. Es wird daher vorgeschlagen, dass die Beschichtung ein Medikament aufweist. Zur Vorbeugung gegen eine Entzündung können Silberionen in die Beschichtung eingebunden werden.

Die Beschichtung kann bei einem Implantat wie insbesondere einem implantierten künstlichen Gefäß langsam absorbiert werden. Vorteilhaft ist es, wenn sie nach ein bis 30 Tagen und vorzugsweise nach 2 bis 14 Tagen auf maximal 10 % der ursprünglichen Menge resorbiert wird. Als Meßmethode eignet sich ein in vivo Modell.

## Patentansprüche

1. Struktur zur Herstellung von Gefäßprothesen mit stellenweise miteinander verklebten Fasern, die eine Durchlässigkeit für Luft zwischen 0,5 ml/min*cm² und 1,5 ml/min*cm² aufweisen, wobei die Struktur ein Vlies aufweist und das Vlies biokompatible Eigenschaften besitzt,
***dadurch gekennzeichnet, dass*** sie eine mindestens einseitige Beschichtung aufweist, die die Durchlässigkeit der Struktur auf unter 0,2 ml/min*cm² reduziert.

2. Struktur nach Anspruch 1, ***dadurch gekennzeichnet, dass*** die Fasern aus Polyurethan bestehen.

3. Struktur nach einem der vorhergehenden Ansprüche, ***dadurch gekennzeichnet, dass*** die Fasern zu einem Zylinder verklebt sind und die Beschichtung an der Zylinderinnenseite angeordnet ist.

4. Struktur nach einem der vorhergehenden Ansprüche, ***dadurch gekennzeichnet, dass*** die Beschichtung Gelatine aufweist.

5. Struktur nach einem der vorhergehenden Ansprüche, ***dadurch gekennzeichnet, dass*** die Beschichtung Kollagene aufweist.

6. Struktur nach einem der vorhergehenden Ansprüche, ***dadurch gekennzeichnet, dass*** die Beschichtung Fibrin aufweist.

7. Struktur nach einem der vorhergehenden Ansprüche, ***dadurch gekennzeichnet, dass*** die Beschichtung ein Medikament aufweist.

8. Struktur nach einem der vorhergehenden Ansprüche, ***dadurch gekennzeichnet, dass*** die Beschichtung nach ein bis 30 Tagen und vorzugsweise nach 2 bis 14 Tagen auf maximal 10 % der ursprünglichen Menge resorbiert wird.

## Claims

1. Structure for manufacturing vascular prostheses, having fibers that are adhesively bonded in certain places and that have a permeability for air between 0.5 ml/min*cm² and 1.5 ml/min*cm², wherein the structure comprises a non-woven material and the non-woven material has biocompatible properties,
***characterized in that*** it has at least a single-sided coating, which reduces the permeability of the structure to below 0.2 ml/min*cm².

2. Structure according to claim 1, ***characterized in that*** the fibers are made of polyurethane.

3. Structure according to one of the preceding claims, ***characterized in that*** the fibers are adhesively bonded to form a cylinder and the coating is arranged at the inner surface of the cylinder.

4. Structure according to one of the preceding claims, ***characterized in that*** the coating comprises gelatine.

5. Structure according to one of the preceding claims, ***characterized in that*** the coating comprises collagens.

6. Structure according to one of the preceding claims, ***characterized in that*** the coating comprises fibrin.

7. Structure according to one of the preceding claims, ***characterized in that*** the coating comprises a medicament.

8. Structure according to one of the preceding claims, ***characterized in that*** the coating is reabsorbed after one to 30 days and preferably after 2 to 14 days up to maximally 10% of the original amount.

## Revendications

1. Structure pour la fabrication de prothèses de vaisseaux avec des fibres collées entre elles par endroits et avec une perméabilité à l'air entre 0,5 ml/min*cm² et 1,5 ml/min*cm², la structure présentant un non-tissé possédant des propriétés biocompatibles
**caractérisée en ce qu'**elle présente un revêtement au moins unilatéral qui réduit la perméabilité de la structure à moins de 0,2 ml/min*cm².

2. Structure selon la revendication 1, **caractérisée en ce que** les fibres sont en polyuréthane.

3. Structure selon l'une des revendications précédentes **caractérisée en ce que** les fibres sont collées de façon à former un cylindre et que le revêtement est disposé sur l'intérieur du cylindre.

4. Structure selon l'une des revendications précédentes **caractérisée en ce que** le revêtement contient de la gélatine.

5. Structure selon l'une des revendications précédentes **caractérisée en ce que** le revêtement contient du collagène.

6. Structure selon l'une des revendications précédentes **caractérisée en ce que** le revêtement contient de la fibrine.

7. Structure selon l'une des revendications précédentes **caractérisée en ce que** le revêtement contient un médicament.

8. Structure selon l'une des revendications précédentes **caractérisée en ce que** le revêtement est résorbé après 1 à 30 jours et qu'il est résorbé de préférence à un maximum de 10% de la quantité d'origine après 2 à 14 jours.
